(19) Europäisches Patentamt
European Patent Office
Office européen des brevets

(11) Numéro de publication : **0 043 327**
**B1**

(12) **FASCICULE DE BREVET EUROPÉEN**

(45) Date de publication du fascicule du brevet : **18.01.84**

(21) Numéro de dépôt : **81401043.5**

(22) Date de dépôt : **29.06.81**

(51) Int. Cl.³ : **B 01 F 17/00**, A 61 K 9/50, A 61 K 7/00, C 10 M 3/10, B 01 D 19/04

(54) **Procédé d'obtention de dispersions stables dans une phase aqueuse d'au moins une phase liquide non miscible à l'eau et dispersions correspondantes.**

(30) Priorité : **01.07.80 FR 8014657**
**24.09.80 FR 8020499**

(43) Date de publication de la demande :
**06.01.82 Bulletin 82/01**

(45) Mention de la délivrance du brevet :
**18.01.84 Bulletin 84/03**

(84) Etats contractants désignés :
**BE CH DE FR GB IT LI**

(56) Documents cités :
**FR-A- 2 221 122**
**FR-A- 2 278 321**
**FR-A- 2 298 318**
**FR-A- 2 315 991**
**FR-A- 2 408 387**
**CHEMICAL ABSTRACTS, vol. 85, no. 4 26 juillet 1976, pages 258, abrégé 25402a Columbus, Ohio, US**

(73) Titulaire : **L'OREAL Société anonyme dite :**
**14, Rue Royale**
**F-75008 Paris (FR)**

(72) Inventeur : **Zabotto, Arlette**
**24, rue Sarrette**
**F-75014 Paris (FR)**
Inventeur : **Griat, Jacqueline**
**11,Quai de la Baronnie**
**F-94480 Ablon (FR)**
Inventeur : **Handjani, Rose-Marie**
**1, place Saint-Sulpice**
**F-75006 Paris (FR)**
Inventeur : **Vanlerberghe, Guy**
**Rue du Général de Gaulle**
**Montjay-la-Tour F-77000 Villevaude (FR)**
Inventeur : **Ribier, Alain**
**16, rue Caffarelli**
**F-75003 Paris (FR)**

(74) Mandataire : **Peuscet, Jacques**
**3, Square de Maubeuge**
**F-75009 Paris (FR)**

**EP 0 043 327 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé d'obtention de dispersions stables dans une phase aqueuse d'au moins une phase liquide non miscible à l'eau et dispersions correspondantes

La présente invention concerne un procédé d'obtention de dispersions stables dans une phase aqueuse d'au moins une phase liquide non miscible à l'eau.

On sait que, lorsque l'on mélange par agitation mécanique, par exemple au moyen d'un ultra-disperseur, une phase liquide non miscible à l'eau dans une phase aqueuse, la stabilité de la dispersion requiert le plus souvent l'addition d'un agent émulsifiant, dont les molécules vont s'adsorber à la surface des gouttelettes de la phase liquide non miscible à l'eau en formant une sorte de membrane continue, qui empêche le contact direct de deux gouttelettes voisines, au cours d'un choc par exemple. Les gouttelettes de phase liquide non miscible à l'eau peuvent contenir des substances solubles en milieu organique. Or, il peut être désirable de réaliser des dispersions qui contiennent, dans des microréservoirs analogues à ceux constitués par les gouttelettes sus-mentionnées, des substances hydrosolubles susceptibles d'avoir une action coordonnée avec celle des substances liposolubles contenues dans les gouttelettes.

On sait, d'autre part, réaliser des dispersions aqueuses de sphérules lipidiques, comme décrit dans FR-A-2 221 122 ET FR-A-2 315 991. Ces sphérules lipidiques ont une structure constituée de plusieurs feuillets lipidiques sensiblement concentriques séparés les uns des autres par des couches de phase aqueuse ; ces sphérules peuvent servir à encapsuler, dans les compartiments aqueux compris entre les couches lipidiques, des substances actives hydrosolubles.

Selon l'invention, on a constaté que l'on pouvait stabiliser des gouttelettes de certains liquides non miscibles à l'eau par la présence, dans la même dispersion, de sphérules lipidiques du type susmentionné. Cette constatation est particulièrement surprenante pour l'homme de l'art car, s'il était possible de penser que les lipides amphiphiles constitutifs des sphérules pouvaient jouer le rôle d'un émulsifiant en venant s'adsorber à la surface des gouttelettes de liquide non miscible à l'eau, par contre, on devait s'attendre au fait qu'une telle stabilisation de ces gouttelettes entraîne la destruction des feuillets concentriques des sphérules. Au contraire, on a constaté que les sphérules constituées de feuillets concentriques de lipides amphiphiles stabilisent les gouttelettes de ces liquides non miscibles dans la dispersion globale et, qu'inversement, les goutelettes de liquide non miscible à l'eau contribuent à la stabilité des sphérules lipidiques. Les dispersions ainsi obtenues ont une stabilité remarquable dans les conditions normales de stockage ; les sphérules gardent leur intégrité, ce qui est d'autant plus surprenant que les liquides organiques ont une action solvante vis-à-vis des lipides amphiphiles dont les sphérules sont constituées.

Les dispersions obtenues par le procédé selon l'invention sont donc constituées par une phase continue aqueuse, dans laquelle sont maintenues en suspension, d'une part, des gouttelettes de certains liquides non miscibles et, d'autre part, des sphérules de feuillets concentriques lipidiques ; on pense que les gouttelettes sont maintenues en suspension par les sphérules adsorbées à leur surface. La formation et la stabilité de telles dispersions dépendent, bien entendu, d'une part, de la nature du liquide non miscible à disperser, d'autre part, de la nature des amphiphiles formant la paroi des sphérules et, enfin, des conditions de mise en œuvre du procédé.

La présente invention a, en conséquence, pour objet, un procédé d'obtention de dispersions stables dans une phase aqueuse D d'au moins une phase liquide L non miscible à l'eau, constituée par au moins un produit pris, dans le groupe formé par les hydrocarbures, les carbures halogénés, les polysiloxanes, les esters d'acides minéraux, les éthers ou polyéthers, caractérisé par le fait que l'on prépare une dispersion dans la phase aqueuse D, de sphérules ayant un diamètre moyen compris entre 0,025 et 5 microns constituées de feuillets lipidiques sensiblement concentriques encapsulant entre eux une phase aqueuse E, les lipides constitutifs des feuillets étant des amphiphiles ioniques ou non-ioniques susceptibles de former dans l'eau une phase lamellaire, que l'on réalise le mélange de cette dispersion et de la (ou des) phase(s) liquide(s) L et que l'on soumet l'ensemble à une agitation mécanique pour disperser la (ou les) phase(s) L dans la phase D en gouttelettes ayant un diamètre moyen compris entre 0,1 micron et quelques microns.

Dans un mode préféré de mise en œuvre, pour réaliser le mélange de la dispersion de sphérules ou la (ou les) phase(s) liquide(s) L, on ajoute ladite (ou lesdites) phase(s) liquide(s) L dans la dispersion de sphérules ; chaque phase liquide L non miscible à l'eau est constituée d'un ou plusieurs composés ayant un volume moléculaire supérieur à 200 $cm^3$/mole ; de préférence, on disperse dans la phase D une phase liquide L unique.

Pour réaliser la dispersion dans la phase aqueuse D des sphérules lipidiques, on peut utiliser n'importe lequel des procédés connus et décrits. On peut, par exemple, utiliser le procédé qui consiste à dissoudre les lipides dans un solvant volatil, à former un film mince de lipides sur les parois d'un flacon par évaporation du solvant, à introduire dans ledit flacon la phase aqueuse E à encapsuler et à agiter le mélange mécaniquement jusqu'à l'obtention de la dispersion de sphérules à la taille désirée ; dans ce cas, les phases aqueuses D et E sont nécessairement identiques. On utilise, de façon préférée, le procédé décrit dans le brevet français 2 315 991, qui consiste à former une phase lamellaire plane par introduction de la phase aqueuse à encapsuler E dans les lipides liquides, à une température légèrement supérieure à la température de fusion des lipides, à ajouter ensuite à la phase lamellaire obtenue une phase aqueuse

de dispersion D, qui peut être identique ou non à la phase aqueuse E, et à agiter énergiquement, par exemple mécaniquement, pour obtenir le passage de la phase lamellaire plane à une dispersion, dans la phase aqueuse D, de sphérules lipidiques encapsulant la phase aqueuse E. Selon les moyens utilisés pour réaliser la dispersion (ultra-disperseur et/ou ultrasons) et selon le temps d'agitation (de 15 mn à quelques heures), on obtient des sphérules, dont le diamètre moyen varie de 0,025 à 5 microns environ.

La dispersion de la (ou des) phase(s) liquide(s) L non miscible(s) à l'eau est avantageusement réalisée à l'aide d'un ultra-disperseur à une température voisine de la température ambiante, ce qui présente un avantage important sur le plan de l'économie, pour la stabilité des constituants de la composition (en particulier, s'ils sont volatils ou oxydables) et pour la sécurité. Le diamètre moyen des gouttelettes de liquide L obtenues varie de 0,1 à quelques microns.

Les lipides utilisés pour la préparation des sphérules sont des amphiphiles d'origine naturelle ou synthétique, ioniques ou non-ioniques, comportant par molécule une ou plusieurs longue(s) chaîne(s) hydrocarbonée(s) et un ou plusieurs groupement(s) hydrophile(s) pris parmi les groupes hydroxyle, étheroxyde, carboxyle, phosphate, amine et ammonium.

Parmi les amphiphiles ioniques, on préfère utiliser les phospholipides naturels (par exemple, la lécithine d'œuf ou de soja ou la sphingomyéline), les phospholipides de synthèse (par exemple, la dipalmitoyl-phosphatidylcholine ou la lécithine hydrogénée), les composés cationiques ou quaternaires (par exemple, le chlorure ou le bromure de didodécyl- ou distéaryl-diméthylammonium) ; on peut aussi utiliser les composés amphotères et les composés anioniques.

Parmi les amphiphiles non ioniques, on préfère utiliser :

1) les éthers de polyglycérol linéaires ou ramifiés de formules respectives

et

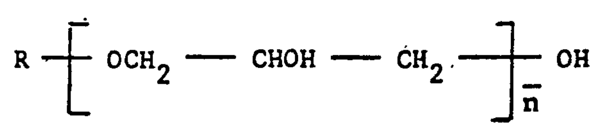

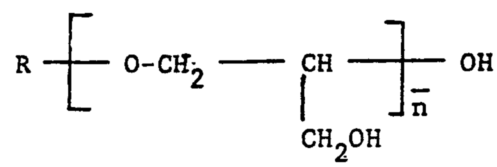

n̄ représentant une valeur statistique moyenne et étant un entier compris entre 1 et 6, R étant une chaîne aliphatique linéaire ou ramifiée, saturée ou insaturée, de 12 à 30 atomes de carbone, des radicaux hydrocarbonés des alcools de lanoline ou les restes hydroxy-2-alkyl des α-diols à longue chaîne ;

2) les alcools gras polyoxyéthylénés ; les stérols polyoxyéthylénés ;

3) les esters de polyols, oxyéthylénés ou non ;

4) les glycolipides d'origine naturelle ou synthétique, par exemple les cérébrosides ;

5) les éthers ou esters de polyols comportant deux groupes alkyl à longue chaîne (au moins à 8 atomes de carbone) comme résidu hydrophobe.

Divers additifs peuvent être associés aux composés lipidiques destinés à former les sphérules en vue de modifier la perméabilité ou la charge superficielle desdites sphérules. On peut citer, à cet égard, l'addition éventuelle des alcools et des diols à longue chaîne, des stérols, par exemple le cholestérol, des amines à longue chaîne et de leurs dérivés ammonium quaternaires, des dihydroxyalkylamines, des amines grasses polyoxyéthylénées, des esters d'aminoalcool à longue chaîne ainsi que leurs sels et dérivés ammonium quaternaires, des esters phosphoriques d'alcools gras et en particulier les esters phosphoriques d'alcools gras, par exemple le dicétylphosphate, des alkylsulfates, par exemple le cétylsulfate de sodium et de certains polymères, tels que les polypeptides et les protéines.

Pour constituer la dispersion de sphérules lipidiques, on utilise de 2 à 10 % de lipides par rapport au poids total de la dispersion de sphérules destinée à être mélangée avec la phase liquide L. Dans un mode de mise en œuvre préféré, les phases aqueuses D et E sont isoosmotiques, et dans la mise en œuvre la plus simple, les phases aqueuses D et E sont identiques.

Pour constituer une phase liquide L, on peut avantageusement choisir parmi les hydrocarbures l'hexadécane et l'huile de paraffine et parmi les carbures halogénés, la perfluorotributylamine et le perfluorodécahydronaphtalène.

Selon un mode de mise en œuvre avantageux, on introduit la phase liquide L dans la dispersion de sphérules dans une proportion allant de 2 à 70 % en poids par rapport au poids de la dispersion de sphérules. On préfère introduire la phase liquide L dans la dispersion de sphérules dans une proportion comprise entre 20 et 2 000 % environ par rapport au poids d'amphiphile(s) contenu(s) dans la dispersion de sphérules.

La présente invention a également pour objet les dispersions stables obtenues par le procédé ci-dessus défini. Le domaine d'application de ces dispersions est très large, puisqu'il réunit le domaine des émulsions de différents types et des dispersions de sphérules de lipides ioniques ou non-ioniques. Les sphérules lipidiques peuvent servir à encapsuler, entre leurs feuillets lipidiques concentriques, des couches de phase aqueuse contenant des substances actives hydrosolubles ; par ailleurs, les couches lipidiques des sphérules peuvent contenir des substances organiques actives. Parmi les substances actives hydrosolubles, on peut citer les composés organiques ou minéraux ayant une activité biologique, microbicide, fongicide, insecticide, vitaminique, photosensible, les médicaments, les réactifs chimiques, les catalyseurs, les colorants, les complexants, les gaz ($O_2$, $CO_2$). Parmi les substances actives liposolubles, on peut citer les antibiotiques et les anti-oxydants.

Dans les dispersions selon l'invention, la présence des gouttelettes de phase liquide L constitue un avantage supplémentaire, car lesdites gouttelettes peuvent contenir des substances solubles en milieu organique, dont l'activité peut être identique ou différente de celle des substances emprisonnées dans les sphérules, la différence entre les deux groupes de substances tenant essentiellement à des solubilités opposées. La phase liquide L elle-même peut, par ses fonctions de solvant ou de véhicule, permettre d'apporter sur un site d'application une substance active : à titre d'exemple, on peut indiquer que les composés perfluorés sont transporteurs d'oxygène et de gaz carbonique, ce qui permet d'envisager de les utiliser comme substituts sanguins. Dans certains cas, la phase liquide L pourra jouer le rôle de lubrifiant, d'agent d'étalement, d'agent de nettoyage, ou d'agent de lustrage. Lorsque son poids moléculaire est relativement faible, la volatilité de la phase liquide L est telle qu'elle s'élimine après dépôt, ce qui peut être très avantageux dans des processus de traitement ou de revêtement de surface. La phase liquide L peut également contenir un polymère, un oligomère, un prépolymère ou un monomère ; elle peut aussi contenir des charges ou des additifs tels que des colorants, des opacifiants ou des gélifiants.

La phase aqueuse continue de la dispersion selon l'invention peut, elle-même, contenir des substances dissoutes analogues ou différentes des substances contenues dans les couches aqueuses ou lipidiques des sphérules et dans la phase liquide L.

On comprend que les sphérules fonctionnent comme un premier type de microréservoirs, qui libèrent lentement les substances hydrosolubles et liposolubles emprisonnées. Par ailleurs, les gouttelettes de phase liquide L constituent un deuxième type de microréservoirs, dont les composés dissous peuvent s'échanger avec un substrat organique, éventuellement celui d'un être vivant. Les actions des sphérules et de la phase liquide L peuvent se combiner, se compléter ou donner lieu à une synergie. Par exemple, la libération des substances actives contenues dans les sphérules peut être accélérée par action conjointe de la température et du pouvoir solvant de la phase liquide L, ce qui peut être utile pour procéder à une opération de polymérisation de monomères ou à une opération de réticulation.

Pour mieux faire comprendre l'objet de l'invention, on va en décrire maintenant, à titre purement illustratif et non limitatif, plusieurs exemples de mise en œuvre.

Exemple 1

Première étape : Préparation de la dispersion de sphérules

Dans un pot en acier inoxydable, on pèse :

— 4,275 g de produit de formule générale :

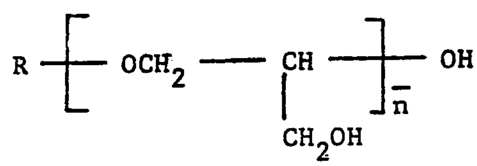

formule dans laquelle R est un radical hexadécyl et $\bar{n}$ a une valeur statistique moyenne égale à 3 ;
— 4,275 g de cholestérol ;
— 0,45 g de dicétyl-phosphate.

On porte ce mélange à une température d'environ 110 °C jusqu'à obtenir une phase lipidique liquide et claire, que l'on refroidit alors à la température de 90 °C. On ajoute 22,5 g d'une solution aqueuse de glucose 0,5 M. On agite doucement à l'aide d'une spatule jusqu'à obtenir une phase apparemment homogène.

On refroidit à la température de 70 °C et on ajoute 68,5 g de la même solution aqueuse de glucose 0,5 M. On soumet le tout à l'agitation donnée par un ultradisperseur ILA modèle CX 1020 tournant à 25 000 tours/mn pendant 10 mn. On refroidit ensuite à 40 °C.

Deuxième étape : Introduction de la phase liquide L non miscible à l'eau

A 100 g de la dispersion de sphérules obtenue dans la première phase, on ajoute 12.5 g de

perfluorodécahydronaphtalène. On soumet ce mélange pendant 5 mn à l'agitation donnée par le même ultradisperseur que celui utilisé à la fin de la première phase du présent exemple.

On obtient ainsi une dispersion stable dont les sphérules ont une dimension moyenne inférieure à 1 micron et dont les gouttelettes ont une dimension moyenne inférieure à 1 micron. Cette dispersion peut être utilisée comme substitut du sang.

En vue d'étudier les caractéristiques de cette dispersion, on détermine la quantité de glucose encapsulée par les sphérules, que l'on utilise comme stabilisateur du perfluorodécahydronaphtalène. Dans ce but, on détermine le gonflement G des lipides constitutifs des sphérules. Si l'on désigne par PS le poids de solution aqueuse de glucose encapsulée et PL le poids de lipides, le gonflement G est calculé par la formule :

$$G = PS/(PS + PL)$$

Pour effectuer cette détermination, étant donné que l'on connaît le poids de lipides PL utilisé, il faut déterminer le poids PS. On place 5 g du produit final dans un sac de dialyse en collodion, que l'on place contre 200 g d'une solution aqueuse de chlorure de sodium à 1,5 % (isoosmotique) sous agitation. On dose la quantité de glucose non encapsulée par les vésicules dans le milieu salin extérieur après le temps d'équilibre de la dialyse (24 heures). On en déduit la quantité de glucose encapsulée PS et l'on calcule que l'on a G = 86 %.

A titre de comparaison, on détermine le gonflement des sphérules avant leur utilisation comme stabilisateur d'huile par dialyse de 5 g de la dispersion obtenue à la fin de la première phase de cet exemple contre 200 g d'une solution isoosmotique de chlorure de sodium. On trouve un gonflement G = 87 %.

## Exemple 2

Première étape : Préparation de la dispersion de sphérules

Dans un flacon en verre, on dissout dans 20 ml de chloroforme :

— 6,75 g de lécithine de soja commercialisée sous le nom « Epikuron 200 » par la société « Lukas Meyer » ;
— 1,8 g de cholestérol ;
— 0,45 g de dicétyl-phosphate.

On lyophilise cette solution à l'aide d'un appareil Virtis modèle 1020. On ajoute au mélange lipidique anhydre obtenu après lyophilisation 91 g d'une solution aqueuse de glucose 1M. On laisse gonfler ce mélange pendant 2 heures à la température de 40 °C sous atmosphère d'azote. On le soumet pendant 10 mn à l'agitation donnée par l'ultradisperseur défini à l'exemple 1, puis on refroidit à la température ambiante. On obtient ainsi une dispersion de sphérules.

Deuxième étape : Introduction de la phase liquide L non miscible à l'eau

On ajoute 12,5 g de perfluorodécahydronaphtalène à 100 g de la dispersion de sphérules obtenue dans la première phase. On soumet ce mélange pendant 5 mn à la température ambiante à l'agitation donnée par l'ultradisperseur utilisé dans la première phase. On obtient une dispersion liquide, qui contient des sphérules ayant un diamètre inférieur à 1 micron et des gouttelettes de perfluorodécahydronaphtalène ayant un diamètre moyen inférieur à 1 micron. Cette dispersion peut être utilisée comme substitut du sang.

On a déterminé le gonflement des sphérules utilisées pour la stabilisation du perfluorodécahydronaphtalène. On a trouvé un gonflement G = 74 %. Le taux de fuite sur 5 jours du glucose encapsulé est de 36 %. Cependant, il n'a pas été possible de déterminer directement le gonflement des sphérules avant dispersion de l'huile ; on a constaté, en effet, que le prélèvement de 5 g de la dispersion obtenue à la fin de la première phase, lorsqu'il est placé en dialyse contre 200 g d'une solution aqueuse de chlorure de sodium à 3 % (isoosmotique), se détériore très rapidement : les sphérules perdent leur intégrité pour reformer une phase visqueuse colmatant le sac de dialyse. Il apparaît donc que, dans cet exemple, les gouttelettes de perfluorodécahydronaphtalène stabilisent les sphérules obtenues dans la première phase. Le gonflement des sphérules avant dispersion d'huile a été déterminé par une autre méthode, à des concentrations en lipides et en glucose plus basses et après filtration sur colonne de gel ; le gonflement trouvé est G = 74 %. Le taux de fuite sur 5 jours du glucose encapsulé est de 45 %. On voit donc que la stabilité de l'encapsulation est améliorée par la présence des gouttelettes du liquide L non miscible à l'eau.

Le procédé utilisé pour l'obtention en deux étapes des dispersions selon l'invention pour les exemples, qui vont suivre, est identique à celui qui a été précédemment décrit dans les exemples 1 et 2, de sorte que les exemples, qui vont suivre, mentionnent uniquement les produits mis en œuvre et les

quantités correspondantes pour chacune des deux étapes de chaque exemple. Les exemples mentionnent également, le cas échéant, le gonflement des sphérules avant et après dispersion d'huile.

Exemple 3

Première étape : Préparation de la dispersion de sphérules

On met en œuvre les produits suivants :

— Produit de formule générale :

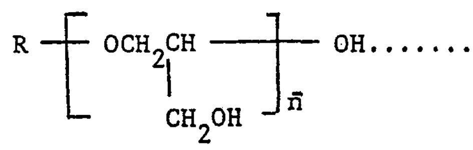 4,275 g

formule dans laquelle R est un radical hexadecyl et n̄ a une valeur statistique moyenne égale à 3 ;

| | |
|---|---|
| — β-Sitostérol | 4,275 g |
| — Dicétyl-phosphate | 0,45 g |
| — Glucose | 8,19 g |
| — Eau | 82,81 g |

On obtient ainsi une dispersion de sphérules.

Deuxième étape : Introduction de la phase liquide L non miscible à l'eau

On ajoute dans la dispersion de sphérules obtenue à la première étape 12,5 g d'une huile de silicone commercialisée par la société « DOWCORNING » sous la référence « DOW 344 ».

Le taux de gonflement des sphérules avant dispersion de l'huile de silicone est de 87 % ; le gonflement des sphérules après dispersion de l'huile de silicone est de 85 %. Le diamètre moyen des sphérules dans la dispersion obtenue est de 1 micron ; le diamètre moyen des gouttelettes d'huile de silicone dans la dispersion obtenue est de 2 microns.

La dispersion obtenue peut être utilisée comme agent anti-mousse.

Exemple 4

Première étape : Préparation de la dispersion de sphérules

On met en œuvre les produits suivants :

— Produit de formule générale :

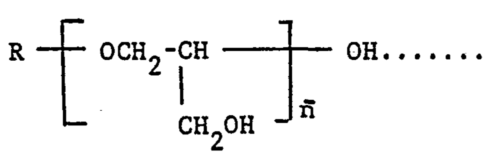 3,8 g

formule dans laquelle R est un radical hexadecyl et n̄ a une valeur statistique moyenne égale à 3 :

| | |
|---|---|
| — Cholestérol | 3,8 g |
| — Dicétyl-phosphate | 0,4 g |
| — Oléate de cholestérol | 0,8 g |
| — Eau | 91,2 g |

On obtient une dispersion de sphérules.

Deuxième étape : Introduction de la phase liquide L non miscible à l'eau

On ajoute à 100 g de la dispersion de sphérules obtenue dans la première étape 40 g de perfluorodécahydronaphtalène. On obtient une dispersion, dans laquelle les sphérules ont un diamètre moyen inférieur à 1 micron et les gouttelettes de perfluorodécahydronaphtalène un diamètre moyen inférieur à 1 micron.

La dispersion ainsi obtenue peut être utilisée comme substitut du sang.

Exemple 5

Première étape : Préparation de la dispersion de sphérules

On met en œuvre les produits suivants :

| | |
|---|---|
| — Lécithine de soja commercialisée par la société « Lukas Meyer » sous le nom « Epikuron 145 » | 9 g |
| — Eau distillée | 91 g |

On obtient une dispersion de sphérules.

Deuxième étape : Introduction de la phase liquide L non miscible à l'eau

Dans 100 g de la dispersion de sphérules obtenue à la première étape, on ajoute 12,5 g d'hexadécane. On obtient ainsi une dispersion dans laquelle les sphérules ont un diamètre moyen de 0,5 micron et les gouttelettes d'hexadécane un diamètre moyen de 2 microns. Cette dispersion peut être utilisée comme lubrifiant pour textiles.

Exemple 6

Première étape : Préparation de la dispersion de sphérules

On met en œuvre les produits suivants :

| | |
|---|---|
| — Dipalmitoyl-lécithine synthétique | 8,5 g |
| — Cholestérol | 1,0 g |
| — Dicétyl-phosphate | 0,5 g |
| — Glucose | 0,1 g |
| — NaCl | 0,054 g |
| — KCl | 0,032 g |
| — $MgCl_2$ | 0,007 g |
| — $CaCl_2$ | 0,010 g |
| — $NaH_2PO_4$ | 0,009 6 g |
| — $Na_2CO_3$ | q.s. pH 7,44 |
| — Eau distillée | q.s 110 g |

On obtient une dispersion de sphérules.

Deuxième étape : Introduction de la phase liquide L non miscible à l'eau

A 100 g de la dispersion de sphérules obtenue dans la première étape, on ajoute 30 g de perfluorotributylamine.

On obtient ainsi une dispersion selon l'invention, dont les sphérules ont un diamètre moyen inférieur à 1 micron et les gouttelettes de perfluorotributylamine un diamètre moyen inférieur à 1 micron. Une telle dispersion peut être utilisée comme substitut du sang.

Exemple 7

Première étape : Préparation de la dispersion de sphérules

On met en œuvre les produits suivants :

| | |
|---|---|
| — Sel de sodium de la N(alkyl(suif))-N(dodecyl)-N-(N',N'-diéthylamino-éthyl)asparagine (décrit dans le brevet français 1 397 231) | 4,95 g |
| — Cholestérol | 3,6 g |
| — Dicétyl-phosphate | 0,45 g |
| — Glucose | 8,19 g |
| — Eau | 82,81 g |

On obtient une dispersion de sphérules.

Deuxième étape : Introduction de la phase liquide L non miscible à l'eau

A 100 g de la dispersion de sphérules obtenue dans la première étape, on ajoute 11 g de perfluorotributylamine. On mesure que le gonflement des sphérules avant et après la deuxième étape est de 78 %.

On obtient une dispersion selon l'invention dans laquelle les sphérules ont un diamètre moyen inférieur à 1 micron et les gouttelettes de perfluorotributylamine un diamètre inférieur à 1 micron. Une telle dispersion peut être utilisée comme substitut du sang.

Exemple 8

Première étape : Préparation de la dispersion de sphérules

On met en œuvre les produits suivants :

| | |
|---|---|
| — Chlorure de dioctadécyl-diméthylammonium | 3 g |
| — Eau distillée | 97 g |

On obtient une dispersion de sphérules.

Deuxième étape : Introduction de la phase liquide L non miscible à l'eau

A 100 g de la dispersion obtenue dans la première étape, on ajoute 12,5 g d'hexadécane. On obtient une dispersion selon l'invention dans laquelle les sphérules ont un diamètre moyen de 1 micron et les gouttelettes d'hexadécane un diamètre moyen de 2 microns.

Cette dispersion est utilisable comme lubrifiant pour textiles.

Exemple 9

Première étape : Préparation de la dispersion de sphérules

On met en œuvre les produits suivants :

| | |
|---|---|
| — Phytostérols oxyéthylénés à répartition statistique de valeur moyenne égale à 5 (produit commercialisé par la société « HENKEL » sous le nom de « GENEROL 115 E5 ») | 6,75 g |
| — Cholestérol | 2,25 g |
| — Eau distillée | 91,0 g |

On obtient une dispersion de sphérules.

Deuxième étape : Introduction de la phase liquide L non miscible à l'eau

A 100 g de la dispersion obtenue dans la première étape, on ajoute 25 g d'huile de paraffine. On obtient une dispersion selon l'invention, dans laquelle les sphérules ont un diamètre moyen de 0,5 micron et les gouttelettes d'huile de paraffine un diamètre moyen de 1 micron.

Cette dispersion est utilisable comme lubrifiant intestinal.

Il est bien entendu que les modes de mise en œuvre ci-dessus décrits ne sont aucunement limitatifs et pourront donner lieu à toutes modifications désirables, sans sortir pour cela du cadre de l'invention.

**Revendications**

1. Procédé d'obtention de dispersions stables dans une phase aqueuse D d'au moins une phase liquide L non miscible à l'eau constituée par au moins un produit pris dans le groupe formé par les hydrocarbures, les carbures halogénés, les polysiloxanes, les esters d'acides minéraux, les éthers ou polyéthers, caractérisé par le fait que l'on prépare une dispersion, dans la phase aqueuse D, de sphérules ayant un diamètre moyen compris entre 0,025 et 5 microns, constituées de feuillets lipidiques sensiblement concentriques encapsulant entre eux une phase aqueuse E, les lipides constitutifs des feuillets étant des amphiphiles ioniques ou non-ioniques suceptibles de former dans l'eau une phase lamellaire, que l'on réalise le mélange de cette dispersion et de la (ou des) phase(s) liquide(s) L et que l'on soumet l'ensemble à une agitation pour disperser la (ou les) phase(s) L dans la phase D en gouttelettes ayant un diamètre moyen compris entre 0,1 et quelques microns.

2. Procédé selon la revendication 1, caractérisé par le fait que, pour réaliser le mélange de la dispersion de sphérules et de la (ou des) phase(s) liquide(s) L, on ajoute la phase L dans la dispersion de sphérules.

3. Procédé selon l'une des revendications 1 ou 2, caractérisé par le fait que chaque phase liquide L

est constituée d'un ou plusieurs composés ayant un volume moléculaire supérieur à 200 $cm^3$/mole.

4. Procédé selon l'une des revendications 1 à 3, caractérisé par le fait que l'on disperse dans la phase aqueuse D une phase liquide L unique.

5. Procédé selon l'une des revendications 1 à 4, caractérisé par le fait que, pour obtenir une dispersion de sphérules dans la phase aqueuse D, on forme une phase lamellaire plane par introduction de la phase aqueuse E dans les lipides liquides, on ajoute ensuite la phase aqueuse D et on agite énergiquement pour réaliser la dispersion de sphérules désirée.

6. Procédé selon l'une des revendications 1 à 5, caractérisé par le fait que, pour obtenir la dispersion finale après addition de la (ou des) phase(s) liquide(s) L, on agite mécaniquement à une température voisine de la température ambiante.

7. Procédé selon l'une des revendications 1 à 6, caractérisé par le fait que, comme lipide destiné à constituer les feuillets des sphérules, on choisit au moins un amphiphile d'origine naturelle ou synthétique, ionique ou non-ionique, comportant par molécule une ou plusieurs longue(s) chaîne(s) hydrocarbonée(s) et un ou plusieurs groupement(s) hydrophile(s) pris parmi les groupes hydroxyle, étheroxyde, carboxyle, phosphate, amine et ammonium.

8. Procédé selon la revendication 7, caractérisé par le fait que, comme amphiphile ionique, on choisit au moins un produit pris dans le groupe formé par les phospholipides naturels, tels que la lécithine d'œuf ou de soja et la sphingomyéline, les phospholipides de synthèse, tels que la dipalmitoyl-phosphatidylcholine ou la lécithine hydrogénée, les composés cationiques ou quaternaires, tels que le chlorure ou le bromure de didodécyl- ou distéaryl-diméthylammonium, les composés amphotères et les composés anioniques.

9. Procédé selon la revendication 7, caractérisé par le fait que, comme amphiphile non-ionique, on choisit au moins un composé pris dans le groupe formé par :

1) les éthers de polyglycérol linéaires ou ramifiés de formules respectives :

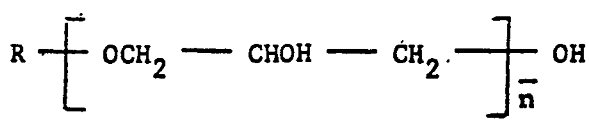

et

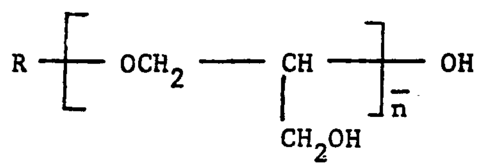

$\bar{n}$ représentant une valeur statistique moyenne et étant un entier compris entre 1 et 6, R représentant une chaîne aliphatique linéaire ou ramifiée, saturée, ou insaturée, contenant 12 à 30 atomes de carbone, des radicaux hydrocarbonés des alcools de lanoline ou les restes hydroxy-2 alkyle des $\alpha$-diols à longue chaîne ;

2) les alcools gras polyoxyéthylénés ou les stérols polyoxyéthylénés ;

3) les esters de polyols, oxyéthylénés ou non ;

4) les glycolipides d'origine naturelle ou synthétique, par exemple les cérébrosides ;

5) les éthers ou esters de polyols comportant deux groupes alkyles ayant au moins 8 atomes de carbone, comme résidu hydrophobe.

10. Procédé selon l'une des revendications 1 à 9, caractérisé par le fait que l'on ajoute aux amphiphiles destinés à former les sphérules, au moins un additif destiné à modifier la perméabilité ou la charge superficielle des sphérules à former.

11. Procédé selon la revendication 10, caractérisé par le fait que l'additif ajouté aux amphiphiles est pris dans le groupe formé par les alcools et diols à longue chaîne, les stérols, les amines à longue chaîne et leurs dérivés ammonium quaternaires, les dihydroxyalkylamines, les amines grasses polyoxyéthylénées, les esters d'amino-alcool à longue chaîne ainsi que leurs sels et dérivés ammonium quaternaires, les esters phosphoriques d'alcools gras, les alkylsulfates, les polypeptides et les protéines.

12. Procédé selon l'une des revendications 1 à 11, caractérisé par le fait que l'on utilise, pour constituer la dispersion de sphérules, de 2 à 10 % en poids d'amphiphile(s) par rapport au poids total de la dispersion de sphérules à obtenir.

13. Procédé selon l'une des revendications 1 à 12, caractérisé par le fait que les phases aqueuses D et E sont isoosmotiques.

14. Procédé selon la revendication 13, caractérisé par le fait que les phases aqueuses D et E sont identiques.

15. Procédé selon l'une des revendications 1 à 14, caractérisé par le fait que l'on mélange la phase

liquide L à la dispersion de sphérules dans une proportion allant de 2 à 70 % en poids par rapport au poids de la dispersion de sphérules.

16. Procédé selon la revendication 15, caractérisé par le fait que l'on introduit la phase liquide L dans la dispersion de sphérules dans une proportion allant de 20 à 2 000 % en poids par rapport au poids d'amphiphile(s) contenu(s) dans la dispersion de sphérules.

17. Dispersion stable dans une phase aqueuse D d'au moins une phase liquide L non miscible à l'eau, caractérisée par le fait qu'elle est obtenue par le procédé selon l'une des revendications 1 à 16.

## Claims

1. Process for obtaining stable dispersions, in an aqueous phase D, of at least one water-immiscible liquid phase L consisting of at least one product taken from the group comprising hydrocarbons, halogenohydrocarbons, polysiloxanes, mineral acid esters, ethers or polyethers, characterised in that a dispersion is prepared, in the aqueous phase D, of small spheres having an average diameter of between 0.025 and 5 microns and consisting of essentially concentric, lipid layers encapsulating an aqueous phase E between them, the lipids constituting the layers being ionic or non-ionic amphiphiles capable of forming a lamellar phase in water, in that this dispersion and the liquid phase (or phases) L are mixed and in that the mixture is agitated in order to disperse the phase (or phases) L in the phase D in the form of droplets having an average diameter of between 0.1 and a few microns.

2. Process according to Claim 1, characterised in that, to mix the dispersion of spheres with the liquid phase (or phases) L, the phase L is added to the dispersion of spheres.

3. Process according to one of Claims 1 or 2, characterised in that each liquid phase L consists of one or more compounds having a molecular volume of more than 200 $cm^3/mol$.

4. Process according to one of Claims 1 to 3, characterised in that a single liquid phase L is dispersed in the aqueous phase D.

5. Process according to one of Claims 1 to 4, characterised in that, to obtain a dispersion of spheres in the aqueous phase D, a plane lamellar phase is formed by introducing the aqueous phase E into the liquid lipids, the aqueous phase D is then added and the mixture is agitated vigorously to produce the desired dispersion of spheres.

6. Process according to one of Claims 1 to 5, characterised in that, to obtain the final dispersion after addition of the liquid phase (or phases) L, the mixture is agitated mechanically at a temperature in the region of ambient temperature.

7. Process according to one of Claims 1 to 6, characterised in that, as the lipid intended for forming the layers of the spheres, at least one ionic or non-ionic amphiphile of natural or synthetic origin is chosen which contains, per molecule, one or more long hydrocarbon chains and one or more hydrophilic groups taken from amongst hydroxyl, ether, carboxyl, phosphate, amine and ammonium groups.

8. Process according to Claim 7, characterised in that, as the ionic amphiphile, at least one product is chosen from the group comprising natural phospholipids such as egg lecithin or soya lecithin and sphingomyelin, synthetic phospholipids such as dipalmitoyl-phosphatidylcholine or hydrogenated lecithin, cationic or quaternary compounds such as didodecyldimethylammonium or distearyl-dimethylammonium chloride or bromide, amphoteric compounds and anionic compounds.

9. Process according to Claim 7, characterised in that, as the non-ionic amphiphile, at least one compound is chosen from the group comprising

1) linear or branched polyglycerol ethers of the respective formulae :

$$R - \left[ OCH_2 - CHOH - CH_2 \right]_{\bar{n}} - OH$$

and

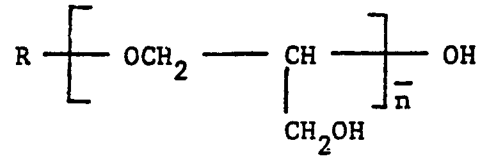

n̄ representing an average statistical value and being an integer between 1 and 6 and R representing a linear or branche saturated or unsaturated aliphatic chain containing 12 to 30 carbon atoms, hydrocarbon radicals of lanoline alcohols or 2-hydroxyalkyl radicals of long-chain α-diols ;

2) polyoxyethyleneated fatty alcohols or polyoxyethyleneated sterols ;

3) oxyethyleneated or non-oxyethyleneated polyol esters ;

4) glycolipids of natural or synthetic origin, for example cerebrosides ; and

5) ethers or esters of polyols containing, as the hydrophobic residue, two alkyl groups having at least 8 carbon atoms.

10. Process according to one of Claims 1 to 9, characterised in that at least one additive intended for modifying the permeability or the surface charge of the spheres to be formed is added to the amphiphiles intended for forming the spheres.

11. Process according to Claim 10, characterised in that the additive added to the amphiphiles is taken from the group comprising long-chain alcohols and diols, sterols, long-chain amines and their quaternary ammonium derivatives, dihydroxyalkylamines, polyoxyethyleneated fatty amines, long-chain aminoalcohol esters and also their salts and quaternary ammonium derivatives, phosphoric acid esters of fatty alcohols, alkyl-sulphates, polypeptides and proteins.

12. Process according to one of Claims 1 to 11, characterised in that, to form the dispersion of spheres, from 2 to 10 % by weight of amphiphile (or amphiphiles) is used, relative to the total weight of the dispersion of spheres to be obtained.

13. Process according to one of Claims 1 to 12, characterised in that the aqueous phases D and E are isoosmotic.

14. Process according to Claim 13, characterised in that the aqueous phases D and E are identical.

15. Process according to one of Claims 1 to 14, characterised in that the liquid phase L is mixed with the dispersion of spheres in a proportion ranging from 2 to 70 % by weight, relative to the weight of the dispersion of spheres.

16. Process according to Claim 15, characterised in that the liquid phase L is introduced into the dispersion of spheres in a proportion ranging from 20 to 2,000 % by weight, relative to the weight of amphiphile (or amphiphiles) contained in the dispersion of spheres.

17. Stable dispersion of at least one water-immiscible liquid phase L in an aqueous phase D, characterised in that it is obtained by the process according to one of Claims 1 to 16.

**Ansprüche**

1. Verfahren zur Herstellung von in einer wässrigen Phase D stabilen Dispersionen aus wenigstens einer nicht mit Wasser mischbaren Phase L, die mindestens aus einem Produkt besteht, das ausgewählt ist unter Kohlenwasserstoffen, halogenierten Kohlenwasserstoffen, Polysiloxanen, Estern von Mineralsäuren, Äthern und Polyäthern, dadurch gekennzeichnet, daß man in der wässrigen Phase D eine Kügelchendispersion mit einem mittleren Durchmesser von 0,025-5 μm herstellt, wobei die Kügelchen aus im wesentlichen konzentrischen, eine wässrige Phase E umhüllenden Lipidplättchen bestehen und wobei die Lipide der Plättchen ionische oder nichtionische Amphiphile sind, die in Wasser eine Lamellenphase bilden können ; diese Dispersion und die flüssig(en) Phase(n) L vermischt und diese Mischung rührt, um die Phase(n) L in der Phase D als Tröpfchen mit einem mittleren Durchmesser zwischen 0,1 und einigen μm zu dispergieren.

2. Verfahren nach Anspruch 1, dadurch gekennzeichnet, daß man, zum Vermischen der Kügelchendispersion mit der (den) flüssigen Phase(n) L, die Phase L in die Kügelchendispersion gibt.

3. Verfahren nach einem der Ansprüche 1 oder 2, dadurch gekennzeichnet, daß jede flüssige Phase L aus einer oder mehreren Verbindungen mit einem Molvolumen größer als 200 $cm^3$/Mol besteht.

4. Verfahren nach einem der Ansprüche 1 bis 3, dadurch gekennzeichnet, daß man in der wäßrigen Phase D eine einzige flüssige Phase L dispergiert.

5. Verfahren nach einem der Ansprüche 1 bis 4, dadurch gekennzeichnet, daß man zwecks Gewinnung einer Kügelchendispersion in der wäßrigen Phase D eine flache Lamellenphase durch Zugabe der wäßrigen Phase E in die flüssigen Lipide bildet, anschließend die wäßrige Phase D zugibt und kräftig rührt, um die gewünschte Kügelchendispersion zu erhalten.

6. Verfahren nach einem der Ansprüche 1 bis 5, dadurch gekennzeichnet, daß man zur Herstellung der endgültigen Dispersion nach Zugabe der flüssigen Phase(n) L mechanisch bei etwa Umgebungstemperatur rührt.

7. Verfahren nach einem der Ansprüche 1 bis 6, dadurch gekennzeichnet, daß man als das, die Blättchen der Kügelchen bildende Lipid wenigstens ein ionisches oder nichtionisches Amphiphil natürlicher oder synthetischer Herkunft wählt, das pro Molekül einen oder mehrere langkettige(n) Kohlenwasserstoff(e) und eine oder mehrere hydrophile Gruppe(n) enthält, ausgewählt unter Hydroxy-, Ätheroxyd-, Carboxyl-, Phosphat-, Amino- und Ammoniumgruppen.

8. Verfahren nach Anspruch 7, dadurch gekennzeichnet, daß man als ionisches Amphiphil wenigstens ein Produkt, ausgewählt unter natürlichen Phospholipiden, wie Eier- oder Sojalecithin und Sphingomyelin ; synthetischen Phospholipiden, wie Dipalmitoylphosphatidylcholin oder hydriertes Lecithin ; kationischen oder quaternären Verbindungen, wie Didodecyl- oder Distearyldimethylammoniumchlorid oder -bromid ; amphoteren und anionischen Verbindungen, verwendet.

9. Verfahren nach Anspruch 7, dadurch gekennzeichnet, daß man als nicht-ionisches Amphiphiles wenigstens eine der Verbindungen verwendet, ausgewählt unter :

1) geradkettigen oder verzweigten Polyglycerinäthern der Formeln :

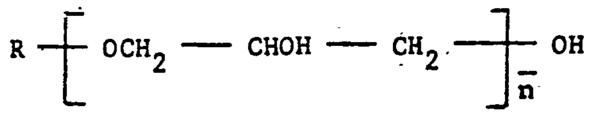

und

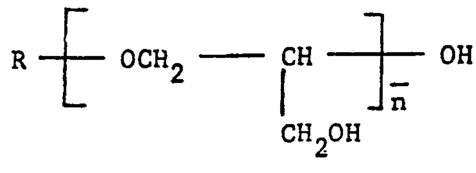

worin

ñ einen statistischen Mittelwert darstellt der eine ganze Zahl zwischen 1 und 6 ist, und R eine geradkettige oder verzweigte, gesättigte oder ungesättigte aliphatische Kette mit 12 bis 30 Kohlenstoffatomen, Kohlenwasserstoffreste von Lanolinalkoholen oder 2-Hydroxy-alkylreste von langkettigen α-Diolen bedeutet ;

2) polyoxyäthylenierten Fettalkoholen oder polyoxyäthylenierten Sterinen ;

3) gegebenenfalls oxyäthylenierten Estern von Polyolyen ;

4) Glycolipiden natürlicher oder synthetischer Herkunft, beispielsweise Cerebrosiden ;

5) Äthern oder Estern von Polyolen, die als hydrophoben Rest 2 Alkylgruppen mit wenigstens 8 Kohlenstoffatomen aufweisen.

10. Verfahren nach einem der Ansprüche 1 bis 9, dadurch gekennzeichnet, daß man zu den Amphiphilen, die zur Bildung der Kügelchen bestimmt sind, wenigstens ein Additiv zur Modifizierung der Permeabilität oder der Oberflächenspannung der zu bildenden Kügelchen gibt.

11. Verfahren nach Anspruch 10, dadurch gekennzeichnet, daß das zu den Amphiphilen gegebene Additiv ausgewählt ist unter langkettigen Alkoholen und Diolen, Sterinen, langkettigen Aminen und deren quaternären Ammoniumderivaten, Dihydroxyalkylaminen, polyoxyäthylenierten Fettaminen, Estern langkettiger Aminoalkohole und deren Salzen und quaternären Ammoniumderivaten, Estern der Phosphorsäure mit Fettalkoholen, Alkylsulfaten, Polypeptiden und Proteinen.

12. Verfahren nach einem der Ansprüche 1 bis 11, dadurch gekennzeichnet, daß man zur Herstellung der Kügelchendispersion 2 bis 10 Gew.-% an Amphiphilem(n), bezogen auf das Gesamtgewicht der zu bildenden Kügelchendispersion, verwendet.

13. Verfahren nach einem der Ansprüche 1 bis 12, dadurch gekennzeichnet, daß die wäßrigen Phasen D und E isoosmotisch sind.

14. Verfahren nach Anspruch 13, dadurch gekennzeichnet, daß die wäßrigen Phasen D und E identisch sind.

15. Verfahren nach einem der Ansprüche 1 bis 14, dadurch gekennzeichnet, daß man die flüssige Phase L zu der Kügelchendispersion in einer Menge von 2 bis 70 Gew.-%, bezogen auf das Gewicht der Kügelchendispersion, mischt.

16. Verfahren nach Anspruch 15, dadurch gekennzeichnet, daß man die flüssige Phase L in die Kügelchendispersion in einer Menge von 20 bis 2 000 Gew.-%, bezogen auf das Gewicht des (der) in der Kügelchendispersion enthaltenen Amphiphilen, gibt.

17. In einer wäßrigen Phase D stabile Dispersion aus wenigstens einer flüssigen, mit Wasser nicht mischbaren Phase L, dadurch gekennzeichnet, daß man sie nach dem Verfahren gemäß einem der Ansprüche 1 bis 16 erhält.